# EUROPÄISCHE PATENTANMELDUNG

(11) **EP 3 610 882 A1**
(43) Veröffentlichungstag der Anmeldung: **19.02.2020**
(21) Anmeldenummer: 19179202.7
(22) Anmeldetag: 07.06.2019
(51) Int. Cl.: A61K 36/9066, A61K 36/61

(54) **PFLANZLICHES KOMBINATIONSPRÄPARAT**

(30) Priorität: 11.06.2018 EP 18177125
(71) Anmelder: Majid, Mohammad S., 5400 Baden (CH)
(72) Erfinder: Majid, Mohammad S., 5400 Baden (CH)
(74) Vertreter: Dittmann, Rolf

(57) **Zusammenfassung**

Beschrieben ist ein pflanzliches Kombinationspräparat, umfassend pflanzliche Inhaltsstoffe aus Granatapfel, Kurkuma und Erd-Burzeldorn.

## Beschreibung

### TECHNISCHES GEBIET

Die vorliegende Beschreibung betrifft ein pflanzliches Kombinationspräparat, wie in den Ansprüchen beschrieben.

### TECHNOLOGISCHER HINTERGRUND

Viele Frauen, insbesondere nach der Menopause, fühlen sich aufgrund von Scheidentrockenheit unwohl und leiden unter häufigen Vaginalinfektionen, sowie unter Libidoverlust infolge des Hormonmangels.

Bei der Behandlung von Scheidentrockenheit werden Arzneimittel auf hormoneller Basis eingesetzt, welche viele Nebenwirkungen mit sich bringen. Des Weiteren kommt Vaseline zur Befettung der äusseren Genitalien zur Anwendung, welche aufgrund der Anwendungsform nur kurzzeitig Linderung verschafft. Es wird ein pflanzliches Produkt angeboten, welches zum Prioritätszeitpunkt der vorliegenden Anmeldung unter dem Namen Delima Feminin im Handel ist, welches die Feuchtigkeit der Scheidenschleimhaut verbessern soll.

### DARSTELLUNG DES GEGENSTANDES DER VORLIEGENDEN BESCHREIBUNG

Wünschenswert ist ein pflanzliches Präparat der eingangs genannten Art, welches zu einer Linderung der beschriebenen Symptomatik beiträgt. Gemäss spezifischerer Aspekte soll eine Kombination aus Inhaltsstoffkomplexen bereitgestellt werden, welche möglichst geringe Nebenwirkungen aufweist. Gemäss weiterer Aspekte sollen ausschliesslich pflanzliche respektive aus Pflanzen gewonnene Inhaltsstoffe zur Anwendung kommen.

Weitere Wirkungen und Vorteile der hier beschriebenen Gegenstände, ob explizit angegeben oder nicht, ergeben sich im Lichte der vorliegenden Beschreibung.

Dies wird mittels des im Anspruch 1 beschriebenen pflanzlichen Kombinationspräparates erreicht.

"Ein" oder "eine" sind im Rahmen der vorliegenden Beschreibung als unbestimmte Artikel und nicht als Zahlwort zu verstehen, sofern nicht explizit auf eine andere Bedeutung, beispielsweise durch die Verwendung von "genau ein" oder "genau eine", hingewiesen wird.

Die Wirkung der Kombination von pflanzlichen Inhaltsstoffen resultiert aus dem Zusammenspiel einer Vielzahl von Inhaltsstoffen einer jeder der genannten Pflanzenzutaten, namentlich der primären und insbesondere der Vielzahl von sekundären Pflanzenstoffen, die in ihrem Gehalt und ihrer Zusammensetzung jeweils für jede der genannten Pflanzen einzigartig und durch diese charakterisiert ist. Beispielsweise berichten Gama et al. in ihrem Artikel «Clinical Assessment of Tribulus terrestris Extract in the Treatment of Female Sexual Dysfunction", Clin Med Insights Womens Health. 2014; 7: 45-50 (Libertas Academia) über Wirkungen von Extrakten aus Tribulus Terrestris. Es hat sich gezeigt, dass bei der gemeinsamen Verwendung von Inhaltsstoffen aus Granatapfel, insbesondere aus Punica Granatum, Kurkuma, insbesondere Curcuma Longa, und Erd-Burzeldorn, insbesondere Tribulus Terrestris, nach ärztlicher Erfahrung des Erfinders synergetische Effekte auftreten.

Insbesondere dann, wenn die pflanzlichen Inhaltsstoffe nach standardisierten Verfahren hergestellt sind, sind die Gehalte wenigstens bestimmter pharmazeutisch bedeutsamer Inhaltsstoffe mit einer Genauigkeit von weniger als ±10 % und insbesondere ±5 %, bezogen auf den nominellen Gehalt des jeweiligen Inhaltsstoffs, bestimmt. Die Angabe der Pflanzenextrakte gibt dem Fachmann somit eine klare Lehre, insbesondere unter Zugrundelegung standardisierter Zubereitungen.

In bestimmten Zubereitungen des pflanzlichen Kombinationspräparates ist ein Komplex aus Inhaltsstoffen aus Granatapfel als pflanzliche Tinktur aus Punica Granatum zugesetzt und/oder ein Komplex aus Inhaltsstoffen aus Kurkuma ist als pflanzliche Tinktur aus Curcuma Longa zugesetzt, wobei die jeweilige Tinktur insbesondere eine nach der Vorschrift HAB 4a hergestellte Urtinktur umfasst. Es kann weiterhin vorgesehen sein, dass ein Komplex aus Inhaltsstoffen aus Tribulus Terriestris als pflanzliche Tinktur zugesetzt wird, wobei die Tinktur insbesondere eine nach den Vorschriften des HAB hergestellte Urtinktur umfasst.

Insbesondere kann jeder der aus einer der genannten Pflanzen gewonnenen Komplexe pflanzlicher Inhaltsstoffe als Potenzierung D1 der jeweiligen Urtinktur nach dem Homöopathisches Arzneibuch HAB enthalten sein.

Die Inhaltsstoffe aus Granatapfel sind in dem Kombinationspräparat beispielsweise in Form einer Tinktur von Punica Granatum UT=D1 nach Herstellvorschrift HAB 4a enthalten. In spezifischeren Beispielen handelt es sich dabei beispielsweise um eine Tinktur von Punica Granatum UT=D1 nach Herstellvorschrift HAB 4a wie sie zum Prioritätszeitpunkt der vorliegenden Anmeldung von der Firma Herbamed AG in 9055 Bühler, Schweiz, hergestellt und vertrieben wird.

Die Inhaltsstoffe aus Kurkuma sind in dem Kombinationspräparat beispielsweise in Form einer Tinktur von Curcuma Longa UT=D1 nach Herstellvorschrift HAB 4a enthalten. In spezifischeren Beispielen handelt es sich dabei beispielsweise um eine Tinktur von Curcuma Longa UT=D1 nach der Herstellvorschrift HAB 4a wie sie zum Prioritätszeitpunkt der vorliegenden Anmeldung von der Firma Herbamed AG in 9055 Bühler, Schweiz, hergestellt und vertrieben wird.

Die Inhaltsstoffe aus Erd-Burzeldorn sind in dem Kombinationspräparat beispielsweise in Form einer nach den Herstellvorschriften des HAB hergestellten Tinktur von Tribulus Terrestris 1DH enthalten. In spezifischeren Beispielen handelt es sich dabei beispielsweise um eine Tinktur nach den Herstellvorschriften des HAB hergestellten Tinktur von Tribulus Terrestris 1DH, wie sie zum Prioritätszeitpunkt der vorliegenden Anmeldung von Laboratoire homeopathique Schmidt-Nagel SA in 1217 Meyrin, Schweiz, hergestellt und vertrieben wird.

Dabei bezeichnet HAB das Homöopathische Arzneibuch in der zum Prioritätszeitpunkt der vorliegenden Anmeldung gültigen Fassung und mit den zum Prioritätszeitpunkt der vorliegenden Anmeldung gültigen Vorschriften.

Von Granatapfel, Punica Granatum, ist bekannt, dass dessen pharmazeutisch relevante Inhaltsstoffe zu einer Verbesserung der Durchblutung der Scheidenschleimhaut beitragen können, was zu einer Feuchtigkeitssteigerung führt. Die ausschliessliche Verabreichung von Punica Granatum kann die durch den im Zuge der Menopause abfallenden Östrogenspiegel verursachte Scheidentrockenheit erfahrungsgemäss nicht vollständig kompensieren. Es hat sich nach ärztlicher Erfahrung des Erfinders gezeigt, dass Erd-Burzeldorn, Tribulus Terrestris, sowohl zu einer verbesserten Durchblutung als auch zu einer Stärkung der Muskulatur beitragen kann. Dies ist in Bezug auf die Muskulatur des Beckenbodens wichtig. Der Östrogenmangel während der Menopause führt zu einer schlechteren Durchblutung der Beckenbodenmuskulatur und schwächt diese. Diesem Problem soll durch die Zugabe von Inhaltsstoffen aus Tribulus Terrestris entgegengewirkt werden. Die Inhaltsstoffe aus Tribulus Terrestris, und insbesondere die darin enthaltenen Saponine, zeigen einen Effekt, der dem von Testosteron ähnelt. Es sei diesbezüglich nochmals auf den oben genannten Artikel von Gama *et al.* verwiesen. Es sind jedoch rein pflanzliche Inhaltsstoffe. Erd-Burzeldorn, insbesondere Tribulus Terrestris, und Granatapfel ergänzen sich in gewünschter Weise. Die Kombination kann nach ärztlicher Erfahrung des Erfinders im Vergleich zur alleinigen Verabreichung von Granatapfel eine signifikante Verbesserung der Durchblutung und Feuchtigkeit der Scheidenschleimhaut unterstützen, da die gestärkte Beckenbodenmuskulatur besser durchblutet wird.

Der Einsatz von Antibiotika und antiinflammatorischen Medikamenten zur Behandlung von vaginalen Infektionskrankheiten verursacht ebenfalls Scheidentrockenheit, da die Schleimhäute angegriffen werden. Um dem Einsatz von Antibiotika sowie antiinflammatorischen Medikamenten vorzubeugen, werden Inhaltsstoffe aus Kurkuma hinzugesetzt. Kurkuma, insbesondere das darin enthaltene Curcumin, zeichnet sich durch seine antiinflammatorische und antioxidative Wirkungsweise aus, was nach ärztlicher Erfahrung des Erfinders den natürlichen Infektionsschutz der Scheide unterstützt.

Aus der Kombination der Inhaltsstoffe der drei genannten Pflanzen Granatapfel, Erd-Burzeldorn und Kurkuma resultiert nach ärztlicher Erfahrung des Erfinders eine synergetische Verbesserung der Wirksamkeit im Vergleich zur isolierten Behandlung mit den Inhaltsstoffen jeder einzelnen Pflanze für sich genommen. Darüber hinaus wird ein natürlicher Schutz vor Infektionen unterstützt.

Das Präparat kann neben den pflanzlichen Tinkturen weiterhin Hilfs- und/oder Trägersubstanzen umfassen. Diese sollen die gewünschte Textur und Handhabbarkeit des Kombinationspräparats herzustellen helfen.

Beispiele für Zusammensetzung des Anteils pflanzlicher Tinkturen sind in den Ausführungsbeispielen und in den Patentansprüchen gegeben.

Die Trägersubstanzen können beispielsweise Gelatine und/oder Glycerol und/oder gereinigtes Wasser, Aqua purificata, umfassen, welche weiterhin insbesondere jeweils den Vorschriften des Europäischen Arzneibuches, Ph.Eur., in der zum Prioritätszeitpunkt der vorliegenden Anmeldung gültigen Fassung entsprechen bzw. nach dessen Vorschriften geprüft wurden. So kann die Gelatine in bestimmten Ausführungsformen Gelatine Golddruck PhEur pulv. sein. Das Glycerol kann in bestimmten Ausführungsformen Glycerol 85% PhEur sein. Das gereinigte Wasser kann in bestimmten Ausführungsformen Aqua purificata PhEur sein.

Das pflanzliche Kombinationspräparat kann als Zäpfchen zum Einführen in die Scheide bereitgestellt sein und gesamthaft 0,5 g oder mehr und 0,7 g oder weniger der pflanzlicher Tinkturen umfassen, bei einer Gesamtmasse des Zäpfchens von beispielsweise 3,0 g bis 4,0 g.

Weitere Ausgestaltungen des vorliegend beschriebenen pflanzlichen Kombinationspräparats erschliessen sich dem Fachmann aus den Patentansprüchen und der weiteren Beschreibung.

Die oben dargestellten Ausgestaltungen der beschriebenen Gegenstände können selbstverständlich untereinander kombiniert werden. Weitere, nicht spezifisch offenbarte Ausführungsformen der Lehre dieses Dokumentes erschliessen sich dem Fachmann ohne Weiteres.

### AUSFÜHRUNGSBEISPIELE

Ein pflanzliches Kombinationspräparat der oben beschriebenen Art wird als Zäpfchen bereitgestellt. Dies stellt die effektivste Verabreichungsform ohne Wirkungsverlust dar. Das Zäpfchen soll weder zu hart sein, aufgrund der sonst bestehenden Verletzungsgefahr bei älteren Frauen, noch zu weich, damit die pflanzlichen Inhaltsstoffe am vorgesehenen Ort bleiben bis sie von der Schleimhaut aufgenommen wurden. Das Kombinationspräparat wird daher als Zäpfchen auf Basis von Glycerin und gereinigtem Wasser als Trägersubstanz für die pflanzlichen Tinkturen bereitgestellt, welches zugleich formstabil und weich ist.

Ein Zäpfchen, das beispielsweise für eine Anwendung am Tag vorgesehen ist, enthält gemäss einem Ausführungsbeispiel gesamthaft 0,58 g der pflanzlichen Tinkturen. Dabei kann vorgesehen sein, dass, zumindest ungefähr, 40% der pflanzlichen Tinkturen, oder 0,23 g, als eine Tinktur von Punica Granatum zugesetzt sind. Es handelt sich dabei beispielsweise um eine Tinktur von Punica Granatum UT=D1 (HAB 4a). Spezifischer handelt es sich um die genannte Tinktur, wie sie zum Prioritätszeitpunkt der vorliegenden Anmeldung von der Firma Herbamed AG in 9055 Bühler, Schweiz, hergestellt und vertrieben wird. Es kann weiterhin vorgesehen sein, dass, zumindest ungefähr, 20% der pflanzlichen Tinkturen, oder 0,12 g, als eine Tinktur von Curcuma Longa zugesetzt sind. Es handelt sich dabei beispielsweise um eine Tinktur von Curcuma Longa UT=D1 (HAB 4a). Spezifischer handelt es sich um die genannte Tinktur, wie sie zum Prioritätszeitpunkt der vorliegenden Anmeldung von der Firma Herbamed AG in 9055 Bühler, Schweiz, hergestellt und vertrieben wird. Es kann weiterhin vorgesehen sein, dass, zumindest ungefähr, 40% der pflanzlichen Tinkturen, oder 0,23 g, als eine Tinktur von Tribulus Terrestris zugesetzt sind. Es handelt sich dabei beispielsweise um eine Tinktur von Tribulus Terrestris 1DH (HAB). Spezifischer handelt es sich um die genannte Tinktur, wie sie zum Prioritätszeitpunkt der vorliegenden Anmeldung von Laboratoire homeopathique Schmidt-Nagel SA in 1217 Meyrin, Schweiz, hergestellt und vertrieben wird. Die pflanzlichen Tinkturen werden mit 0,27 g Gelatine Golddruck PhEur pulv, 2,23 g Glycerol 85% PhEur sowie 0,43 g Aqua purificata PhEur vermischt und als Vaginalzäpfchen geformt. Die angegebenen Mengen gelten für ein Zäpfchen, wobei der Fachmann die für die Herstellung grösserer Chargen und/oder stärker dosierter Zäpfchen notwendigen Mengen selbstverständlich problemlos zu ermitteln vermag.

Obschon der Gegenstand der vorliegenden Beschreibung anhand ausgewählter Ausführungsbeispiele erläutert wurde, sollen diese nicht einer Einschränkung der beanspruchten Erfindung dienen. Die Ansprüche umfassen Ausführungsformen, die nicht explizit dargestellt sind, und Ausführungsformen, die von den gezeigten Beispielen abweichen, sind dennoch von den Ansprüchen gedeckt.

## Patentansprüche

1. Pflanzliches Kombinationspräparat, umfassend pflanzliche Inhaltsstoffe aus Granatapfel, Kurkuma und Erd-Burzeldorn.

2. Pflanzliches Kombinationspräparat gemäss dem vorstehenden Anspruch, wobei die Inhaltsstoffe aus Granatapfel als pflanzliche Tinktur aus Punica Granatum zugesetzt sind und/oder die Inhaltsstoffe aus Kurkuma als pflanzliche Tinktur aus Curcuma Longa zugesetzt sind, wobei die pflanzliche Tinktur insbesondere eine nach der Vorschrift HAB 4a hergestellte Urtinktur umfasst.

3. Pflanzliches Kombinationspräparat nach einem der vorstehenden Ansprüche, wobei die Inhaltsstoffe aus Erd-Burzeldorn als pflanzliche Tinktur aus Tribulus Terriestris zugesetzt sind, wobei die pflanzliche Tinktur insbesondere eine nach der Vorschrift HAB hergestellte Urtinktur umfasst.

4. Pflanzliches Kombinationspräparat gemäss einem der beiden vorstehenden Ansprüche, wobei wenigstes eine der pflanzlichen Tinkturen eine Potenzierung D1 nach HAB einer nach der Vorschrift HAB hergestellten Urtinktur ist.

5. Pflanzliches Kombinationspräparat gemäss einem der vorstehenden Ansprüche, wobei das Kombinationspräparat einen Anteil pflanzlicher Tinkturen umfasst, wobei der genannte Anteil pflanzlicher Tinkturen pflanzliche Tinkturen von Punica Granatum, Curcuma Longa und Tribulus Terrestris umfasst, und das Kombinationspräparat weiterhin einen Anteil Trägersubstanzen umfasst.

6. Pflanzliches Kombinationspräparat gemäss dem vorstehenden Anspruch, wobei der genannte Anteil pflanzlicher Tinkturen aus pflanzlichen Tinkturen von Punica Granatum, Curcuma Longa und Tribulus Terrestris besteht.

7. Pflanzliches Kombinationspräparat gemäss einem der vorstehenden Ansprüche, wobei der Anteil pflanzlicher Tinkturen einen Anteil der Tinktur von Punica Granatum von 35% oder mehr und 45% oder weniger umfasst und/oder zu 35% oder mehr und zu 45% oder weniger aus der Tinktur von Punica Granatum besteht, wobei die Prozent-Angaben als Massen-Prozent bezogen auf den gesamten Anteil pflanzlicher Tinkturen im Kombinationspräparat zu verstehen sind.

8. Pflanzliches Kombinationspräparat gemäss einem der vorstehenden Ansprüche, wobei der Anteil pflanzlicher Tinkturen einen Anteil der Tinktur von Curcuma Longa von 15% oder mehr und 25% oder weniger umfasst, und/oder zu 15% oder mehr und 25% oder weniger aus der Tinktur von Curcuma Longa besteht, wobei die Prozent-Angaben als Massen-Prozent bezogen auf den gesamten Anteil pflanzlicher Tinkturen im Kombinationspräparat zu verstehen sind.

9. Pflanzliches Kombinationspräparat gemäss einem der vorstehenden Ansprüche, wobei der Anteil pflanzlicher Tinkturen einen Anteil der Tinktur von Tribulus Terrestris von 35% oder mehr und zu 45% oder weniger umfasst, und/oder zu 35% oder mehr und zu 45% oder weniger aus der Tinktur von Tribulus Terrestris besteht, wobei die Prozent-Angaben als Massen-Prozent bezogen auf den gesamten Anteil pflanzlicher Tinkturen im Kombinationspräparat zu verstehen sind.

10. Pflanzliches Kombinationspräparat gemäss einem der vorstehenden Ansprüche, wobei der Anteil der Trägersubstanzen Gelatine umfasst.

11. Pflanzliches Kombinationspräparat gemäss einem der vorstehenden Ansprüche, wobei der Anteil der Trägersubstanzen Glycerol 85% umfasst.

12. Pflanzliches Kombinationspräparat gemäss einem der vorstehenden Ansprüche, wobei der Anteil der Trägersubstanzen gereinigtes Wasser umfasst.

13. Pflanzliches Kombinationspräparat gemäss einem der vorstehenden Ansprüche, wobei das Kombinationspräparat als Zäpfchen bereitgestellt ist.

14. Pflanzliches Kombinationspräparat gemäss dem vorstehenden Anspruch, wobei ein Zäpfchen gesamthaft 0,5 g oder mehr und 0,7 g oder weniger der pflanzlichen Tinkturen umfasst.

15. Pflanzliches Kombinationspräparat gemäss einem der beiden vorstehenden Ansprüche, wobei ein Zäpfchen eine Gesamtmasse von 3,0 g oder mehr und 4,0 g oder weniger aufweist.
